# EUROPEAN PATENT APPLICATION

(11) **EP 4 527 409 A1**
(43) Date of publication of application: **26.03.2025**
(21) Application number: 23382951.4
(22) Date of filing: 20.09.2023
(51) Int. Cl.: A61K 41/00, C12N 5/079, C12N 5/077, C12N 5/0775, C12N 5/09

(54) **METHOD FOR PRODUCING THERAPEUTIC EXTRACELLULAR VESICLES FROM MESENCHYMAL STEM CELLS BY DIRECT PULSED ELECTRICAL STIMULATION**

(71) Applicant: Consejo Superior De Investigaciones Científicas, 28006 Madrid (ES); Université de Liège, 4000 Liège (BE); Universidad de Cantabria, 39005 Santander (ES); Institut Químic de Sarrià CETS Fundació Privada IQS, 08017 Barcelona (ES)
(72) Inventor: Mobini, Sahba, Madrid (ES); González Sagardoy, María Ujué, Madrid (ES); Guix Ráfols, Francesc Xavier, Barcelona (ES); Ramos Villar, Ana Victoria, Cantabria (ES); Espuny Camacho, Ira, Liège (BE)
(74) Representative: Pons

(57) **Abstract**

The present invention relates to a method for the production of therapeutic extracellular vesicles (EVs) from Mesenchymal Stem Cells by applying a direct pulsed electrical stimulation to said Mesenchymal Stem Cells. Furthermore, the present invention also discloses the EVs, the composition or the kit comprising them, and their use in regenerative therapy and the delivery of drugs.

## Description

The present invention relates to a method for the production of therapeutic extracellular vesicles from Mesenchymal Stem Cells by applying a direct pulsed electrical stimulation to said Mesenchymal Stem Cells. Furthermore, the present invention also discloses the use of the therapeutic extracellular vesicles so obtained in regenerative therapy and the delivery of drugs. Thus, the present invention relates to the technical field of medicine, particularly, to the regenerative therapy.

### BACKGROUND ART

Extracellular vesicles (EVs) are micro/nano scale, membrane-bound particles that are produced and released by cells into the extracellular space. They play a crucial role in intercellular communication by transferring functional biomolecules such as lipids, proteins, and different types of RNA from one cell to another. Recently, EVs have gained attention as potential therapeutic agents in regenerative medicine due to their ability to deliver therapeutic molecules leading to regeneration or repair of the damaged tissues. The utilization of EVs in regenerative medicine has the potential to revolutionize current treatments for a wide range of diseases, including neurodegenerative disorders, cardiovascular diseases, and cancer and replace conventional cell therapy (due to drawback of cell therapy, see references).

One major challenge in the development of EVs as therapeutic agents is the low yield of production. In general, a 1000 ml of conditioned culturing media yields approximately 10⁹-10¹¹ EVs, an amount that is typically sufficient only for one single test in mice models. This low yield of production has hindered the progress of EV-based therapies, as it is difficult to obtain enough material for preclinical and clinical trials.

Another important obstacle is the lack of control over the content of EVs. EVs are produced by cells through a process that involves the packaging of molecules into the vesicles, that is difficult to predict or control. In addition, the composition of EVs can be influenced by a variety of factors, including the state of the donor cell, and the microenvironment in which the cell resides.

Genetic modification and pre-conditioning with chemical and physical stimuli including mechanical, irradiation and electrical stimulation are recently proposed for increasing the yield of EVs production and their content (also named cargo).

Genetic modification requires cumbersome and strict manufacturing regulations for clinical application and suffer from low efficiency.

Pre-conditioning with chemical stimulation also suffers from the cumbersome and strict manufacturing regulations and low efficiency.

Pre-conditioning with mechanical stimulation lacks control of the process, reproducibility and scalability.

Pre-conditioning with irradiation (Ewing, N. et al. 2023. Pharmaceutical Research 40:833-853; Moertl, S. et al. 2020. International Journal of Molecular Sciences 21 :2336) is hindered by the high cytotoxicity of radiation resulting in low cell viability after treatment and the lack of control in quality of the EVs due to cell stress.

Pre-conditioning with electrical stimulation holds several advantages; it is controllable, automatable, and scalable. Electrical stimulation in range of endogenous electric fields does not compromise the cell viability and function.

For instance, Ewing, N. et al. 2023 (cited *ad supra*)*,* the international patent application WO2019136268A1, Zhang, H. et al. 2023. Cells 12: 875, and Wang, Y. *et al.* 2019 (doi: https://doi.org/10.1101/566448), disclose electrical stimulation methods for producing EVs. Nonetheless, said methods are only applied to electroactive cells such as neurons and cardiac cells. There is no straightforward indication that non-electroactive cells respond in a similar way to electrical stimulation.

Fukuta, T. et al. 2020. (Biochemistry and Biophysics Reports 21, 100713) discloses electrical stimulation using salt bridges electrodes (indirect electrical stimulation) on fibroblast cells in normal and cancerous conditions. They showed that the EVs yield is increased. However, the quality of EVs is unchanged and therefore, no modification on the cargo has taken place, thereby limiting their applications in regenerative therapy.

Moreover, indirect electrical stimulation is mediated by ionic concentration gradients that are slow and complicated to control due to its dependence on chemical reactions. In contrast, the direct electrical stimulation, which is based on direct delivery of electric and ionic charges, is faster and more controllable.

Therefore, it is necessary to provide new methods to enhance the yield of therapeutic EVs production and to control and improve their cargo.

### DESCRIPTION OF THE INVENTION

Electrobiology is an emerging field in regenerative medicine. The intrinsic role of electrical field in interaction with cells and tissues and their extensive diagnostic and therapeutic application have rapidly grown in the field of regenerative medicine for the past two decades. In the present invention, researchers provide a method which is performed at low intensity, low frequency electrical stimulation (ES) in the range of endogenous field that provokes cellular responses such as proliferation, differentiation, and migration. Consequently, electrical stimulation in the range of endogenous electrical filed can be viewed as a tool to alter cell secretome.

The present invention refers to a method of direct pulsed electrical stimulation of Mesenchymal stem cells (MSCs) under specific conditions with metallic electrodes which surprisingly boosts the production of therapeutic Extracellular Vesicles (EVs) (4 to 5 folds production increase) and which surprisingly results in EVs which show improved regenerative properties in comparison with EVs from non-stimulated MSCs or stimulated under different electrical stimulation conditions. This is a genetics-free, biochemical-free and scalable method that makes it advantageous among current established strategies. Furthermore, it is to mention that the electrical stimulation conditions of the method of the present invention do not produce electroporation.

As indicated above, the method of the present invention unexpectedly modifies the cargo of therapeutic EVs obtained from the MSCs so stimulated, giving rise to EVs with an up-regulated expression of proteins involved in neural tissue regeneration. The method disclosed herein increases several proteins related to the biogenesis and trafficking of EVs, that is in agreement with the increase of production of EVs. More importantly, this method surprisingly increases the presence of proteins that are essential in neural plasticity, development, hippocampus-dependent learning and memory, metabolism, and cytoskeleton regulation. These factors are highly beneficial in neural regeneration and survival. This modified cargo confers EVs the unexpected therapeutical properties.

The functionality of these EVs and their regenerative properties are proven via applying them to human brain organoids. Human brain organoids differentiated from human pluripotent stem cells (hPSC) and treated in culture with EVs from electrically stimulated MSCs showed significant acceleration in growth and cell survival compared to those that were treated with natural EVs. These results are strongly supported by quantification of Ki67 (proliferation) and PHH3 (cell division) expression and by measuring the size of organoids (n=8-10). Overall, these results suggest that EVs from electrically stimulated MSCs are pro-regenerative and pro-survival, showing augmented regenerative effects.

The present invention refers additionally to the EVs obtained by the method of the present invention for use in regenerative therapy; for instance, for use in:
- Tissue repair and regeneration: EVs can promote tissue repair and regeneration by stimulating cell proliferation, migration, and differentiation. For example, mesenchymal stem cell-derived EVs have been shown to promote tissue regeneration in animal models of heart, liver, and kidney injury.
- Wound healing: EVs can enhance wound healing by promoting angiogenesis and reducing inflammation. They have been shown to improve skin wound healing in animal models.
- Bone regeneration: EVs can promote bone regeneration by stimulating osteogenic differentiation of stem cells. They have been shown to improve bone healing in animal models of bone defects.
- Cartilage repair: EVs can promote chondrogenic differentiation of stem cells and stimulate cartilage repair. They have been shown to improve cartilage regeneration in animal models of osteoarthritis.
- Neuroprotection: EVs can protect neurons from damage and promote their survival. They have been shown to improve recovery in animal models of stroke and traumatic brain injury.
- Immune modulation: EVs can modulate the immune response by promoting an anti-inflammatory environment and reducing immune cell activation. They have been shown to improve outcomes in animal models of autoimmune diseases and transplant rejection.
- Generating EVs for drug delivery: EVs can be engineered and loaded with drugs. Electrically stimulated EVs can be used for producing EVs for drug delivery engineering.

In view of the foregoing, a first aspect of the present invention relates to a method, hereinafter "method of the invention", for the production of therapeutic extracellular vesicles from Mesenchymal Stem Cells comprising a step (a) of applying a direct pulsed electrical stimulation directly to the cells during a period of 30 minutes to 8 hours per 1 to 10 days with an electrical stimulation device comprising a plurality of metallic electrodes (1), the Mesenchymal Stem Cells located in the cell culture wells of the electrical stimulation device, said cell culture wells comprising cell culture medium, the plurality of metallic electrodes (1) located inside the cell culture wells of the electrical stimulation device and in direct contact with the cell culture medium and/or the Mesenchymal Stem Cells,
the electrical stimulation device producing
   - an electric field of between 10 mV/mm and 100 mV/mm
   - a pulse of an amplitude range between 200 mV and 3 V,
   - and a frequency ranging between 1 Hz and 1000 Hz,
wherein the pulse width is ranging between 1% and 50% of the duration of the pulse and wherein the duration of the pulse is the inverse of the frequency.

As used herein, the term "Extracellular Vesicles" (EVs) refers to small, nano- to microsized lipid bilayer membrane particles that are secreted out from living cells and packed with regulatory biological cargo, such as cytosol molecules, lipids, proteins and nucleic acids. The EVs are classified through different biogenesis pathways into exosomes and microvesicles.

As used herein, the term "Mesenchymal Stem Cells" (MSCs) means a multipotent cell type originally derived from the mesenchyme, being mesenchyme the meshwork of embryonic connective tissue from which all other connective tissues of the body are formed, including cartilage and ultimately bone. Thus, the MSCs are stromal cells that have the ability to self-renew and also exhibit multilineage differentiation. Unlike neurons and cardiac cells, MSCs are non-electroactive cells; therefore, is it not a straightforward assumption that MSCs respond to electrical stimulation in the context of the EVs production and cargo control. MSCs can be isolated from a variety of tissues including, without limiting to, umbilical cord, endometrial polyps, menses blood, bone marrow, adipose tissue, menstrual blood and endometrium etc.

In a particular embodiment of the method of the invention, the MSCs are human adipose MSCs, which is extremely relevant for clinical application.

The method of the invention comprises applying "direct" electrical stimulation to the MSCs with a device comprising a plurality of metallic electrodes directly embedded in the cell culture medium. Direct electrical stimulation functions based on direct delivery of charges to the cells and cell culture medium. Direct electrical stimulation is used in opposition to indirect electrical stimulation: e.g., inductive stimulation with capacitive and/or inductive coupling; electromagnetic coupling; and electrochemical salt-bridges.

Inductive stimulation is far less efficient than direct electrical stimulation, because there is no delivery of charged species to the system. Stimulation with electrochemical salt bridges is mediated by ionic concentration gradients that are slow and complicated to control due to its dependence on chemical reactions. In contrast, the direct electrical stimulation, which is based on direct delivery of electric and ionic charges, is faster, controllable and more efficient.

Therefore, as mentioned before, the method of the invention comprises a step (a) of applying a direct pulsed electrical stimulation for the production of therapeutic extracellular vesicles from Mesenchymal Stem Cells, the Mesenchymal Stem Cells located in the cell culture wells of the electrical stimulation device, said cell culture wells comprising cell culture medium and the plurality of metallic electrodes (1) located inside the cell culture wells of the electrical stimulation device and in direct contact with the cell culture medium and/or the Mesenchymal Stem Cells.

Furthermore, in the context of the present invention, "electrical stimulation" refers to the application of an electrical field of between 10 mV/mm and 100 mV/mm to the MSCs without provoking the electroporation thereof. The electric pulse amplitude is in the range between 200 mV and 3 V, and the frequency ranges between 1 Hz and 1000 Hz, wherein the pulse width is ranging between 1% and 50% of the duration of the pulse and wherein the duration of the pulse is the inverse of the frequency. Preferably, the electrical stimulation device produces a 50 Hz pulse width of at least 500us or a 1Hz pulse width of at least 250 ms. Furthermore, non-pulsed stimulation (i.e., DC stimulation) will not be effective due to ionic screening and electric field cancelation. Excessive voltage beyond 3 V generates harmful reactive oxygen species and water hydrolysis that are detrimental for the cells.

Additionally, it is to be mentioned that the method of the invention comprises applying a direct pulsed electrical stimulation to MSCs during a period of 30 minutes to 8 hours per 1 to 10 days. In a particular embodiment of the method of the invention, the direct pulsed electrical stimulation of step (a) is applied directly to the Mesenchymal Stem Cells during 30 minutes and 90 minutes per 1-10 days. A more preferred duration is 1 hour per day for 3 days. 3 days is an important time point as the cell culture media in MSCs need to be periodically exchanged every 3 days for optimum cell maintenance conditions. Therefore, if the method is used for longer periods, frequent extraction of EVs is required. Besides, un-interrupted stimulation causes evaporation, altering the medium and harming cell viability and function. Therefore, stimulation duration is limited up to 8 hours.

Any electrical stimulation device can be used in the method of the invention to stimulate the MSC.

In particular embodiment of the method of the invention, the electrical stimulation device used comprises
- a cell culture well-plate; and
- an electrode adapted for the electrical stimulation of a cell culture housed in one or more wells of the plate, wherein the electrode comprises at least one material disposed as a substrate and a coating of electrically conductive material comprising platinum.

In particular embodiment of the method of the invention, the electrical stimulation device used comprises
- a cell culture well-plate; and
- an electrode adapted for the electrical stimulation of a cell culture housed in one or more wells of the plate, wherein the electrode comprises at least one material disposed as a substrate and a coating of electrically conductive material comprising platinum nanopillars disposed on said substrate by means of glancing angle deposition (GLAD) with magnetron cathode sputtering.

The electrical stimulation device used in the method of the present invention for applying the pulsed electrical stimulation to the MSCs comprises a plurality of metallic electrodes; said electrodes can be the anode or the cathode of the device.

The term "metallic electrode" refers herein to any electrode made from the bulk metal, shaped as wire, stripe, foil, slab or similar, as well as to any electrode composed of a thin layer of metallic material deposited on any metallic or non-metallic substrate of any kind and shape.

In the present invention, metallic electrodes are placed inside the cell culture vessel, in contact with cells and/or cell medium, and connected to the external source that provides either current or voltage. Metallic electrodes are mechanically more robust than polymer or carbon based. The conductivity properties of metals are superior compared to other materials. Cleaning and sterilization of metallic electrodes is much easier and more efficient than polymer or carbon-based electrodes. Among all metals, those with less reactivity are preferred as they are more biocompatible and resistant to corrosion in the electrochemical processes taking place inside the cell culture vessel.

In a particular embodiment of the method of the invention, metallic electrodes are platinum (Pt) electrodes. Platinum electrodes are preferred for the method of the present invention because
- Platinum is biocompatible, meaning it is well-tolerated by living organisms and does not cause significant adverse reactions or toxicity when in contact with biological systems.
- Platinum is highly stable under various physiological conditions, making it resistant to corrosion and degradation. This stability ensures the long-term functionality and reliability of bioelectrodes (better than gold, titanium, PEDOT and Carbon).
- Platinum exhibits excellent electrical conductivity, allowing efficient electron transfer at the electrode-electrolyte interface. This is important for achieving high-performance bioelectrodes that can accurately detect, measure, or stimulate electrical signals in biological systems. In this regard Pt is much better than carbon electrodes with conductivity of 0.1-4.5 MS/m as Graphite and 0.01-1 MS/m in amorphous state.
- Platinum displays a pseudocapacitive behavior, due to reversible faradaic reactions at the interface (e.g., Pt + H2O ⇆ PtO + 2H+ + 2e- and Pt + H2O + e-⇆ Pt-H + OH-) that boosts safe electron injection to electrolyte.
- Platinum is highly stable and resistant to corrosion, making it suitable for long-term use in demanding electrochemical environments. Carbon electrodes, on the other hand, can be susceptible to degradation and chemical reactions under certain conditions, limiting their applicability in some cases.
- Platinum electrodes can be fabricated with precise control over size, shape, and surface area, allowing for tailored designs to meet specific requirements. Carbon electrodes can also be engineered, but platinum offers more flexibility in terms of shaping and patterning.
- Platinum can be deposited as a stable ultra-thin layer while providing bulk platinum properties that will cost equal or less than a carbon electrode in the bulk form. Moreover, the surface of platinum can be tailored to provide superior electrochemical properties using CVD or sputtering methods, while carbon only used as a bulk material for their application as electrode (i.e., IonOptix Device). Platinum is also more cost effective than IrOx that provides comparable electrochemical properties to platinum.
- Finally, platinum electrodes do not require cleaning after in vitro stimulation, while other materials such as carbon electrodes, do. Without cleaning, carbon electrodes can accumulate biological and chemical residues on their surfaces during use, which can affect their performance and sensitivity over time. Cleaning the electrodes is mandatory to remove these contaminants and restore their functionality. This does not happen with platinum electrodes.

In another preferred embodiment of the method of the invention, said method further comprises a step (b), after step (a), of collecting and harvesting the EVs produced in step (a).

Methods for collecting and harvesting EVs are known to persons skilled in the art. One such example includes differential centrifugation. In another example, the method comprises collecting the spent medium from the stimulated cells and subjecting the medium to centrifugation and filtration.

Further methods for collecting and harvesting include ultra-centrifugation, ultrafiltration, density gradient ultrafiltration, hydrostatic dialysis, size-exclusion chromatography, and precipitation with polymer or protamine.

The centrifugation may be carried out at 2,000 x g, particularly, for a period of time, preferably at least 10-20 minutes. The supernatant produced from centrifugation may be filtered. In a particular embodiment, the pore size of the filter is in the range of about 0.1 pm to 0.45pm. Various filter sizes may be contemplated including 0.1 pm, 0.22 pm and 0.45 pm. Alternatively, the EVs may be isolated/harvested using a commercial kit. Such kits are known in the art and include PureExo^{®} Exosome Isolation Kit, Exosome Isolation kit (Life Technologies, CUSABIO), Exo-Quick (System Bioscience), Exo-Pure 9BioVision, Exo-spin (Cell Guidance System).

As a consequence of putting into practice the method of the invention, the production of therapeutic EVs from the MSCs is increased in comparison with that of non-stimulated MSCs, and the cargo of said EVs is modified, obtaining EVs with improved therapeutic properties, mainly augmented regenerative effects. The increase in the yield of EVs is 4 to 5-fold which is unexpectedly high as the MSCs are not electroactive cells. Even more surprisingly, due to its non-electroactive condition, the content of the EVs produced by MSCs with the method of invention contains a higher number of proteins related to regenerative effects.

Therefore, in another aspect, the present invention relates to therapeutic EVs, hereinafter "EVs of the invention", obtained by the method of the invention, as defined above.

As the skilled person in the art understand, the EVs of the invention can be part of a composition, in particular, a pharmaceutical composition or a cosmetic composition (i.e.: non-therapeutic composition). Thus, in another aspect, the present invention relates to a composition comprising the EVs of the invention (hereinafter "composition of the invention").

The composition of the invention, further to the EVs of the invention, may comprise other compounds such as excipients, carriers, active agents, etc.

According to the conventional techniques known to those skilled in the art, the composition according to the present invention may be formulated with excipient and/or carrier. Thus, in a particular embodiment, the composition of the invention comprises an excipient and/or carrier which, in case of being a pharmaceutical composition, it may be formulated with a pharmaceutically acceptable excipient and/or carrier.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight. When the form of presentation is liquid, the carrier is the diluent. In a particular embodiment, the carrier is pharmaceutically acceptable.

Routes of administration include topical, ocular, nasal, pulmonary, buccal, parenteral (intravenous, subcutaneous, and intramuscular), oral, vaginal and rectal. Administration from implants is also possible. The composition of the invention, in particular the pharmaceutical composition, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Typical carriers include alcohol, water, mineral oil, liquid petrolatum, white petrolatum, glycerin, polyethylene glycol and propylene glycol. Penetration enhancers may be incorporated.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms. The pharmaceutical composition may also be provided in bandages or in sutures or the like.

Biodegradable particles have been shown to be capable of delivering a variety of therapeutic agents. Thus, the present invention also encompasses biodegradable microparticles comprising the EVs of the invention.

The composition of the invention may comprise, further to the EVs of the invention, other therapeutic agents including, but without limiting to, antibiotics, painkillers, chemotherapeutics, hormones, analgesics, anti-inflammatory agents, anticoagulants, anti-hypertensive agents, antibodies, antibody conjugates, proteins, biologically active proteins, fusion proteins, peptides, polypeptides (e.g. cytokine, chemokine, enzyme, hormone etc.), vaccine antigens, blood products, anti-toxins, polynucleotides, and small molecules and combinations of any of the foregoing, or other therapeutic agents useful in the regenerative therapy.

Additionally, the EVs or the composition of the invention can be part of a kit. Thus, in another aspect, the present invention relates to a kit, hereinafter "kit of the invention" comprising the EVs or the composition of the invention.

As use herein, the term "Kit" refers to a product containing the EVs of the invention (or the composition comprising them), which are packaged to enable them to be transported and stored. The kit may further include, without any limitation, buffers, agents to prevent contamination, etc. Suitable materials for packaging the components of the kit include glass, plastic (polyethylene, polypropylene, polycarbonate and the like), bottles, vials, paper, sachets and the like. Additionally, the kits of the invention may contain instructions for simultaneous, sequential or separate use of the different components in the kit. Said instructions may be in the form of printed material or in the form of an electronic medium capable of storing instructions so that they can be read by a subject, such as electronic storage media (magnetic disks, tapes, USB drives and the like), optical media (CD-ROM, DVD) and the like.

Additionally, the EVs or the composition of the invention may also be part of a scaffold. Thus, in another aspect, the present invention relates to a scaffold comprising (i) the EVs of the invention, or (ii) the composition of the invention.

As used herein, the term "scaffold" refers to a three-dimensional structure comprising a material that provides a surface suitable for adherence/attachment and proliferation of cells. A scaffold may further provide mechanical stability and support. A scaffold may be in a particular shape or form so as to influence or delimit a three- dimensional shape or form assumed by a population of proliferating cells. Preferably, the scaffold is a three-dimensional substrate made from a material approved by a health authority, for human use.

Due to the properties of the EVs of the invention as explained at the beginning of the description, in another aspect, the present invention relates to the EVs or the composition of the invention for use as a medicament, particularly, for use in regenerative therapy, hereinafter "use of the invention".

In the context of the present invention, the expression "regenerative therapy" or "regenerative medicine" refers to the process directed to replace or produce tissue or organs that have been damaged by disease, trauma, or congenital issues in order to restore their normal function/activity. Therefore, the EVs of the invention can be used in tissue engineering, wound healing, cellular therapies, and the production of artificial organs. Bearing this in mind, examples of diseases which can be treated with the EVs of the invention include, without limiting to, cancer, stroke, cardiovascular diseases, radiodermitis, erythromelalgia, peripheral artery disease, renal artery stenosis, Buerger's disease, Raynaud's disease, disseminated intravascular coagulation, cerebrovascular disease, acute lymphoblastic leukemia, chronic lymphocytic leukemia, acute myelogenous leukemia, chronic myelogenous leukemia, hairy cell leukemia, T-cell prolymphocytic leukemia, juvenile myelomonocytic leukemia, systemic lupus erythematous, scleroderma, hemolytic anemia, vasculitis, Type 1 diabetes, Graves' disease, rheumatoid arthritis, osteoarthritis, psoriatic arthritis, Goodpasture syndrome, pernicious anemia, myopathy, lyme disease, thrombophilia, coagulopathy, acute traumatic coagulopathy, sensory modulation disorder, atherosclerosis, inflammatory bowel disease, Alzheimer's, ankylosing spondylitis, asthma, Crohn's disease, colitis, dermatitis, diverticulitis, fibromyalgia, hepatitis, nephritis, Parkinson's disease, skin disorders, and ulcerative colitis, or any pathological manifestations that require wound healing or tissue regeneration, shortening healing times, or aiding in resolving the lesion.

In a particular embodiment of the use of the invention, the regenerative therapy is for the treatment of neurovascular diseases including but not limited to peripheral nerve injury, stroke, brain traumatic injury, spinal cord traumatic injury, and Alzheimer's disease.

The term "treatment" as used herein refers to fighting the effects caused as a consequence of the disease or pathological condition of interest (like tissue damage) in a subject (preferably a mammal and, more preferably, a human), including:
i) inhibiting the disease or pathological condition, i.e., stopping its development;
(ii) alleviating the disease or pathological condition, i.e. causing the regression of the disease or pathological condition or its symptoms;
(iii) stabilizing the disease or pathological state.

In another aspect, the present invention relates to the EVs of the invention for use in the delivery of drugs.

### DESCRIPTION OF THE DRAWINGS

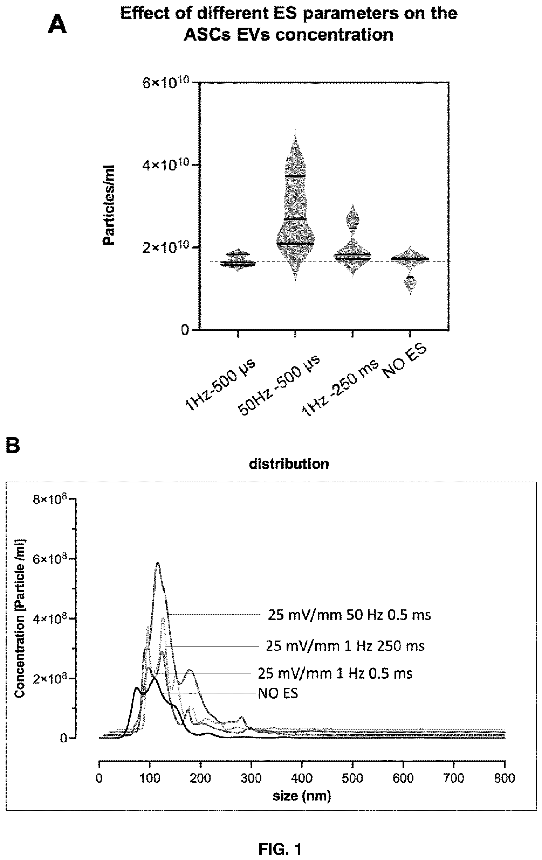

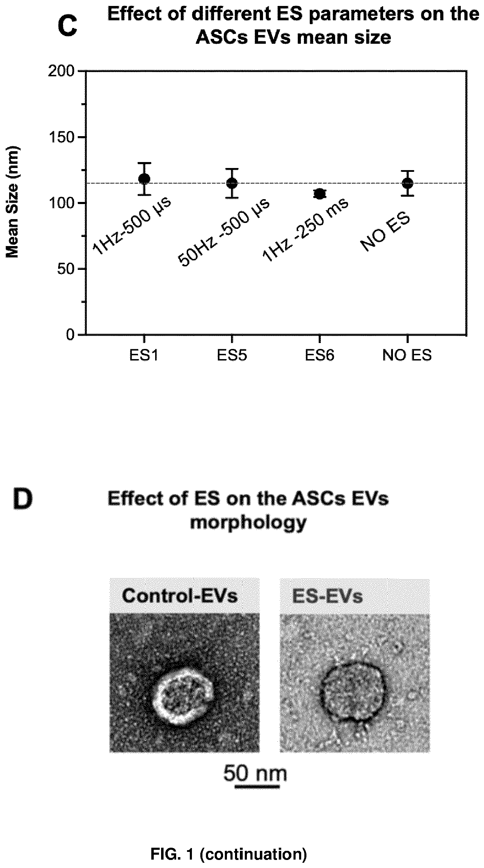

**Figure 1****. Electrical stimulation amplifies the production of EVs in human adipose derived mesenchymal stem/stromal cells (ASCs).** A) Effect of selected electrical stimulation regime (see Table 1) on EVs concentration. B) Mean size of EVs secreted from electrically stimulated ASCs with different protocols and control (NO ES). C) Size distribution of EVs secreted from ASCs treated with different electrical stimulation protocols and control (NO ES). D) Transmission electron microscopy (TEM) images from EVs secreted from electrically stimulated ASCs (right) and control (left).

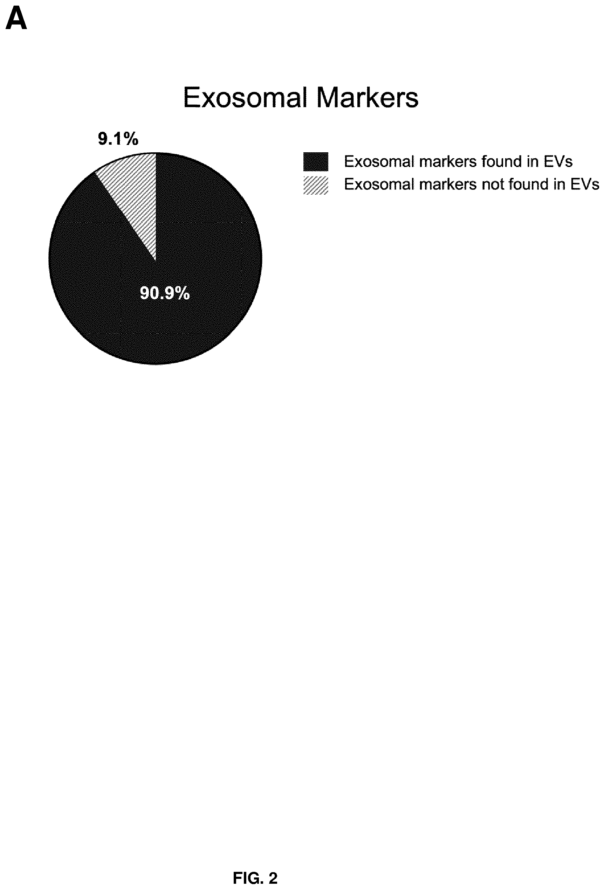

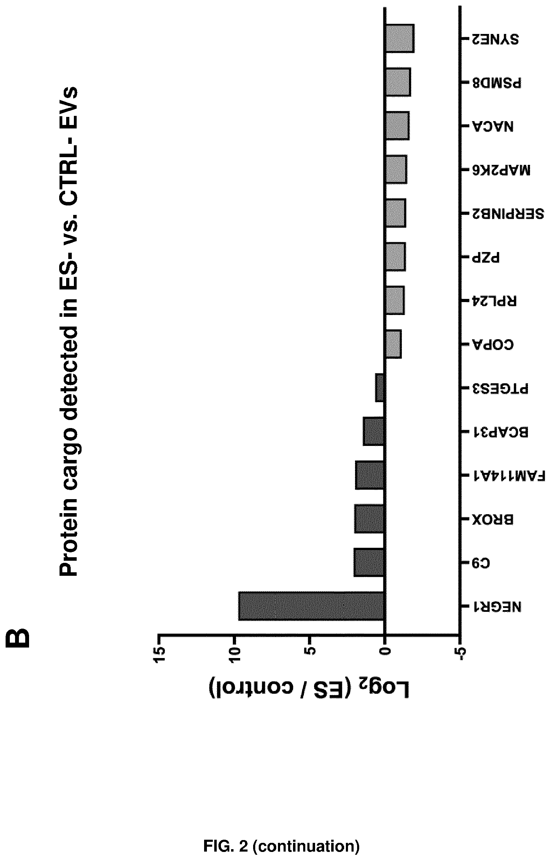

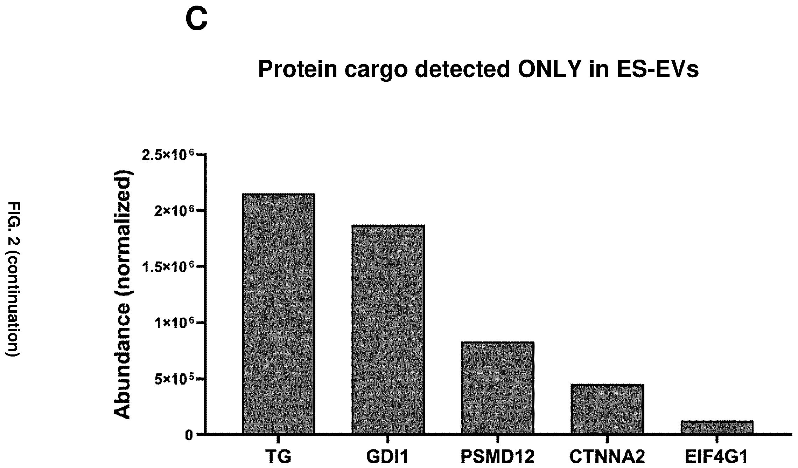

**Figure 2****. Quality of isolated EVs from electrically stimulated and control (NO ES) human adipose derived mesenchymal stem/stromal cells (ASCs) and protein cargo.** A) Amount of exosomal markers detected in isolated EVs compared to standard exosomal marker catalogue (ExoCarta), which is above 90%. B) The abundant protein detected in EVs obtained from electrically stimulated mesenchymal stem cells (ES-EVs) normalized vs non-stimulated MSCs (CTRL-EVs). C) Proteins detected exclusively in ES-EVs.

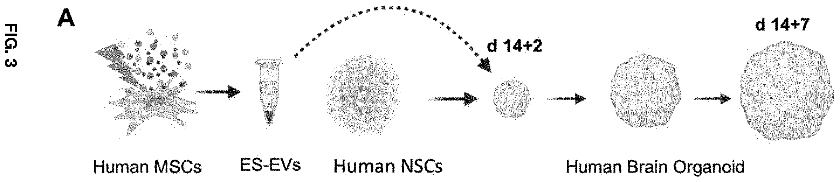

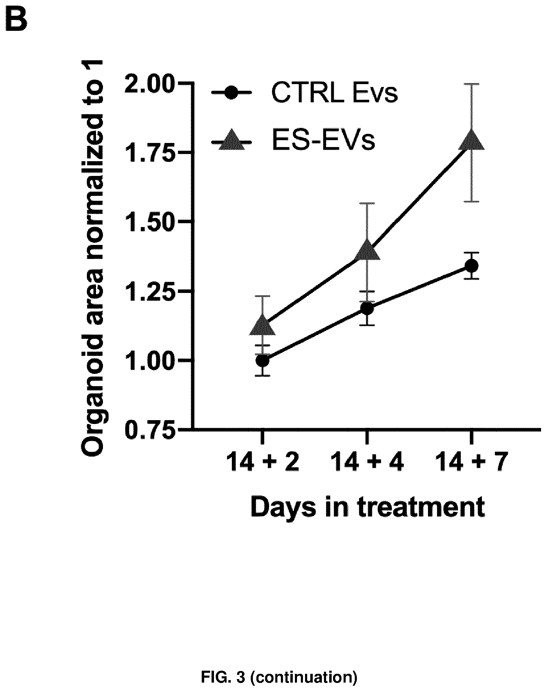

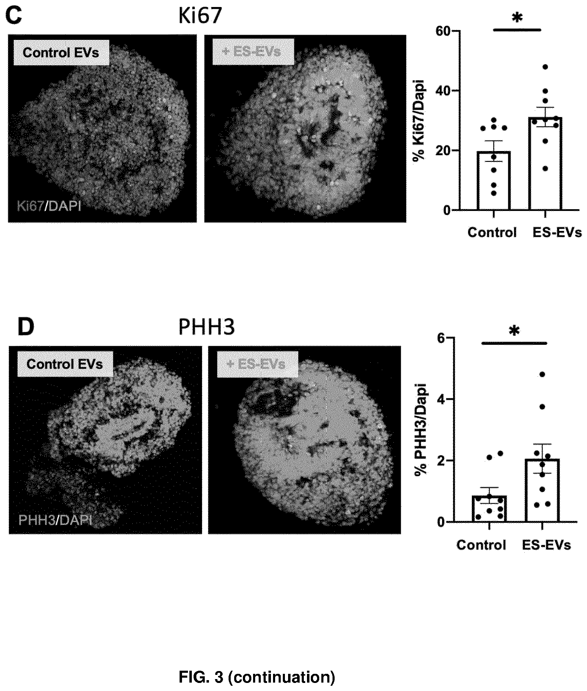

**Figure 3** **EVs from electrically stimulated mesenchymal stem cells increases human brain organoids growth.** A) Schematic summary of the experiment. Human brain organoids are differentiated and treated with EVs obtained from electrically stimulated mesenchymal stem cells (ES-EVs) and from non-stimulated MSCs (CTRL-EVs) as control, and their growth is monitored. B) Organoid area measured from phase-contrast images acquired during 1 week of treatment of human brain organoids with ES-EVs or CTRL-EVs. Organoid area values are normalised to control (CTRL-EVs) brain organoid area. C) Confocal images of Ki67 staining and Ki67 quantification of human brain organoids treated with CTRL-EVs and ES-EVs. ES-EVs treatment significantly increases the expression of Ki67, a proliferative marker. D) Confocal images of PHH3 staining and PHH3 quantification of human brain organoids treated with CTRL-EVs and ES-EVs. ES-EVs treatment increases the expression of PHH3, a mitosis marker, significantly.

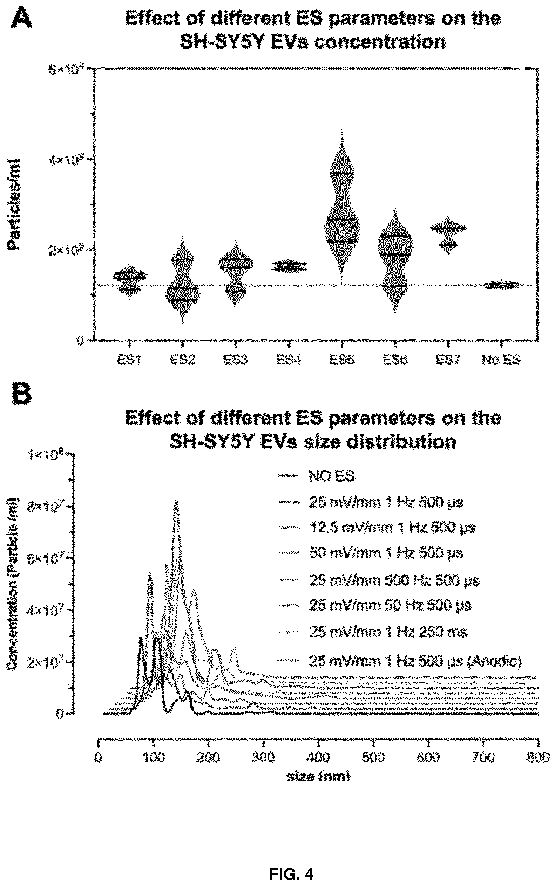

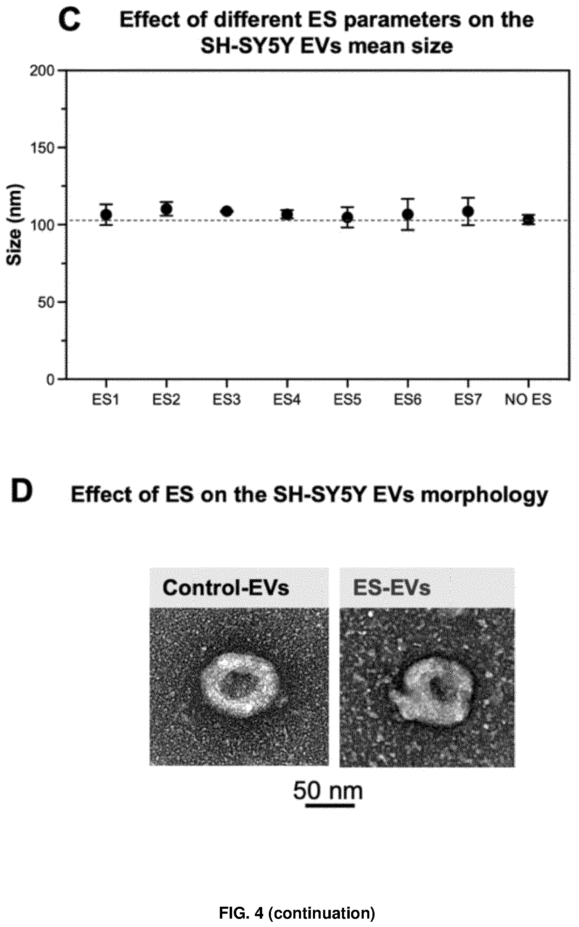

**Figure 4** **Electrical stimulation amplifies EVs production in human Neuroblastoma.** A) Effect of different electrical stimulation regimes (see table 2) on EVs concentration produced by SH-SY5Y neuroblastoma cells. B) Mean size of EVs secreted from electrically stimulated SH-SY5Y cells with different protocols (ES) and control (NO ES). C) Size distribution of EVs secreted from neuroblastoma cells treated with different electrical stimulation protocols and control. D) Transmission electron microscopy (TEM) images from EVs secreted from electrically stimulated SH-SY5Y cells (right) and control (left).

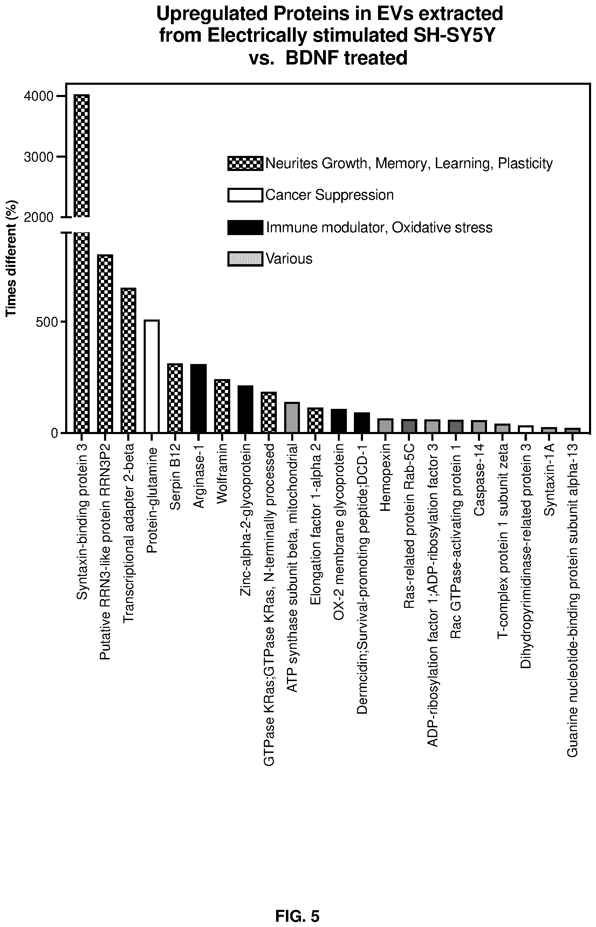

**Figure 5** **Proteomics analysis of EVs obtained from electrical stimulated SH-SY5Y cells compared to EVs obtained from BDNF treated cells.** Several groups of proteins in EVs obtained from electrical stimulated SH-SY5Y cells are upregulated compared to EVs from the cells treated by commercial BDNF. Checker pattern indicates group of protein that are involved in neurite growth, synaptic formation, memory, learning, and plasticity. White indicates protein involved in cancer suppression. Black indicates group of proteins that are anti-inflammatory and immunomodulators, promoting the survival after damage and oxidative stress. Gray indicates proteins with various functions. Overall, the results suggest that the content of EVs from electrically stimulated cells are pro-regenerative and pro-survival.

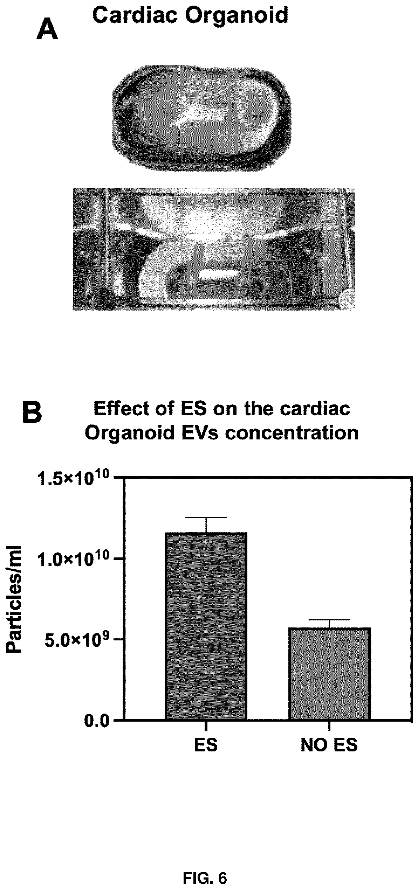

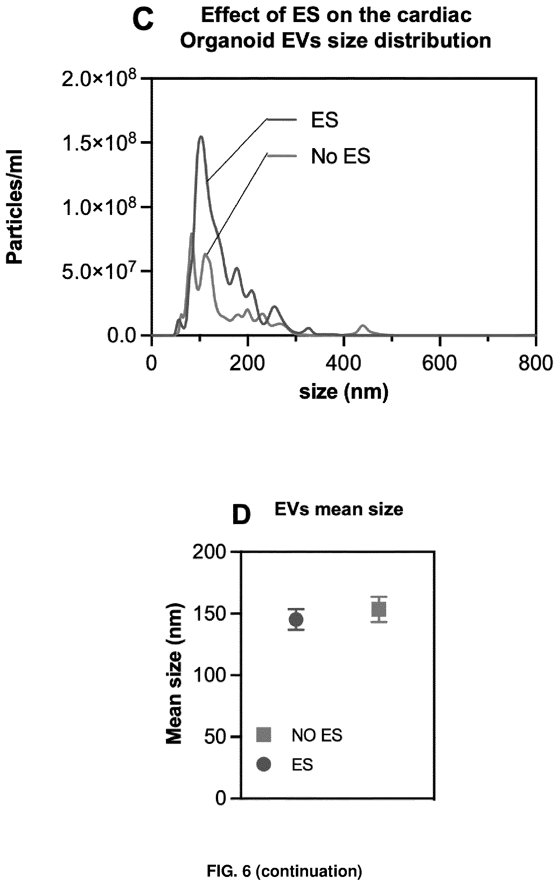

**Figure 6** **Electrical stimulation amplifies EVs production in cardiac organoids.** A) A single well of the 48-well myriamed^{®} plate equipped with mini silicon poles to culture the cardiac organoid (down). Human cardiac organoid developed from primary fibroblasts to study 3D cardiac fibrotic tissue in vitro (up). B) Average concentration of EVs secreted from the cardiac organoids with and without electrical stimulation. C) Size distribution of EVs secreted from cardiac organoids with and without electrical stimulation treatment. D) Mean size of EVs secreted from electrically stimulated and control (NO ES) organoids.

### Examples

### Example 1. Human Mesenchymal Stem/Stromal cells (non-electroactive)

Human adipose tissue derived mesenchymal stem cells (ASCs) were cultured in electrical stimulation chamber at the density of 5 K/cm².

Figure 1. Electrical stimulation amplifies the production of EVs in human adipose derived mesenchymal stem/stromal cells (ASCs). A) Effect of selected electrical stimulation regime (see Table 1) on EVs concentration. B) Mean size of EVs secreted from electrically stimulated ASCs with different protocols and control (NO ES). C) Size distribution of EVs secreted from ASCs treated with different electrical stimulation protocols and control (NO ES). D) Transmission electron microscopy (TEM) images from EVs secreted from electrically stimulated ASCs (right) and control (left).

Selected effective electrical stimulations was tested on ASCs and the results on the extracted EVs are shown in Figure 1.

Electrical stimulation was performed under the conditions included in Table 1. All groups were treated for 1 hour per day, for 3 days. The control group was kept in culture for 3 days with no treatment. All experiments have been carried out at least in three biological replicates.

| **Table 1. Electrical stimulation regimes used in ASCs** | | | | | | | |
|---|---|---|---|---|---|---|---|
| **Regime** | **Graph** | **Frequency** | **Amplitude** | **Field** | **Offset** | **Pulse width** | **Description** |
| ES-01 | 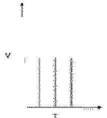 | 1 Hz | 800 mV | ~25 mV/mm | 0 | 500 µs | Anodic Monophasic |
| ES-02 | 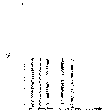 | 50 Hz | 800 mV | ~25 mV/mm | 0 | 500 µs | Anodic Monophasic |
| ES-03 | 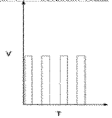 | 1 Hz | 800 mV | ~25 mV/mm | 0 | 250 ms | Anodic Monophasic |

The selected conditions of Table 1 are the result of an optimisation process. These regimes are chosen to be both effective and safe for the cell viability and healthy function. For example, increasing the amplitude of the applied voltage beyond 3 V generates excess amount of reactive oxygen species (as measured in the inventors' lab) and induces the hydrolysis of water (which is the main component of the culture medium) that is detected by the presence of bubbles in the electrode area. Both effects are detrimental for the cells. In another example, changing pulses to continues stimulation removes the effectiveness of the stimulation, due to the ionic screening. This has been shown in inventors' lab by electrochemical analysis and simulation of the equivalent electrical circuit. Finally, un-interrupted stimulation provokes evaporation that consequently changes the composition of the culture medium, which negatively affects cell viability and function. Therefore, the duration of electrical stimulation has been limited to a maximum of the 8 hours.

The cell culture media containing secreted products were collected and subjected to series of centrifugation (300 xg 20 min and 2000 xg 20 min). The collected supernatant was subjected to 100,000 xg ultracentrifugation using 20% sucrose in PBS as cushion for, 3 hours with a wash after 1 hour. A BECKMAN ultracentrifuge and rotor adapted to 38 ml tubes were used.

The particle size distribution and the number of particles in the collected EVs suspension were measured using NTA device (Malvern Panalytical NanoSight NS300 Instrument, UK).

All electrical stimulation conditions increase the concentration of secreted EVs, with the highest increment obtained by 25 mV/mm, 50 Hz pulses of 500 µs. In this case the concentration of the secreted EVs significantly increases from ~1.5×10¹⁰ for non-stimulated to ~ 2.8×10¹⁰ for stimulated cells. (Figure 1 A)

The result of NTA analysis showed that the particle size distribution is the same in all EVs obtained from stimulated and non-stimulated cells. These results are obtained from three individual experiments with 3 technical replicates. Therefore, electrical stimulation does not change the size distribution and average size (around 120 nm) of produced EVs that is comparable with EVs naturally produced by the cells (NO ES).(Figures 1 B and C).

Transmission electron microscopy (TEM) imaging was performed on EVs using TEM (JEM1010, Jeol). The results show that there are no morphological differences between the EVs extracted from electrically stimulated cells and non-stimulated cells. (Figure 1 D)

The protein content of isolated EVs has been analysed by means of mass spectrometry, Protein Identification mode using LC-ESI-MS/MS-HR, Medium Gradient, (Thermo Orbitrap Exploris 240, equipped with FAIMS ion mobility modules and two nano HPLC Ultimate 3000).

Figure 2 Quality of isolated EVs from electrically stimulated and control (NO ES) human adipose derived mesenchymal stem/stromal cells (ASCs) and protein cargo. A) Amount of exosomal markers detected in isolated EVs compared to standard exosomal marker catalogue (Exocarta), which is above 90%. B) The abundant protein detected in EVs obtained from electrically stimulated mesenchymal stem cells (ES-EVs) normalized vs non-stimulated MSCs (CTRL-EVs). C) Proteins detected exclusively in ES-EVs.

It is showed herein that the quality of the isolated EVs meets high standard, both for ES-EVs, and CTRL-EVs. Samples from both groups present over 90% of exosomal markers indexed in Exocarta (e.g., CD81, CD9, CD63, Tsg101, Hsp90, Alix (PDCD6IP), FLOT1 / FLOT 2). This indicates that EVs isolation protocol is highly reliable, and more importantly, EVs from electrically stimulated cells (ES-EVs) are highly pure and do not contain anomalies such as presence of apoptotic bodies and cell debris. This information is in agreement with morphological data presented in Figure 1.

It is also showed that there are at least 6 proteins that are significantly more abundant in the ES-EVs than CTRL-EVs. these proteins are NEGR1, C9, BROX, FAM114A1, BCAP31, and PTGES3. Among these proteins, NEGR1 expressed over 1000 times more in ES-EVs. NEGR1 is related to neural development and synaptic plasticity. This protein is involved in the formation and maintenance of neuronal connections in the brain. BROX, 5 times more expressed in ES-EVs than in CTRL-EVs, plays important role in multivesicular body formation, biogenesis of EVs and EVs trafficking.

Finally, at least 5 proteins with significant abundancy have been detected that are ONLY found in ES-EVs: TG, GDI1, PSMD12, CTNNA2, EIF4G1. TG is a key protein for regulation of metabolism; GDI1 is involved in exosomal biogenesis and vesicular trafficking; PSMD12 has an important role in brain and neural development and its deficiency might lead to neurological disorders; CTNNA2 is related to cytoskeleton regulation through enabling the binding activity of the actine filament; and EIF4G1 controls hippocampus-dependent learning and memory.

Overall, these results indicate that: 1) ES increases the biogenesis of EVs backed up by increase in the related EVs biogenesis and trafficking markers. 2) ES alters the cargo of EVs toward the increase and present of proteins that are essential in neural plasticity, development, hippocampus-dependent learning and memory, metabolism, and cytoskeleton regulation. These factors are highly beneficial in neural regeneration and survival, implying the therapeutic potential application of ES-EVs.

EVs obtained from electrically stimulated human ASCs showed pro-regenerative / proliferation effect on human brain organoids.

The effect of EVs from electrically stimulated mesenchymal stem cells (ES-EVs) was compared with EVs from non-stimulated same cells (CTRL-EVs) on acceleration of brain organoid growth.

Figure 3 EVs from electrically stimulated mesenchymal stem cells increases brain organoid growth. A) Schematic summary of the experiment. Brain organoids are treated with EVs obtained from electrically stimulated mesenchymal stem cells (ES-EVs) and from non-stimulated MSCs (CTRL-EVs) as control and their growth is monitored. B) Organoid area measured by means of imaging during 1 week for brain organoids treated with ES-EVs or CTRL-EVs. C) Ki67 staining in organoids treated by CTRL-EVs and ES-EVs. ES-EVs significantly increases the expression of Ki67, proliferative marker. D) PHH3 staining in organoids treated by CTRLEVs and ES-EVs. ES-EVs increases the expression of PHH3, mitosis marker, significantly.

Briefly, human mesenchymal stem cells from adipose tissue were cultured at density of 2.5K/cm² and stimulated by 25mV/mm pulses of 500µs width and 1 Hz frequency for 1 hour per day for 3 days. Secretome was collected a purified and subjected to centrifugation at 100Kxg for 3 hours. EVs of each 25K cells were resuspended in 100 µL PBS (1 dose).

Human brain organoids differentiated from human pluripotent stem cells for 14 days in vitro. Human brain organoids of 14 days in vitro were treated with 3 doses of either ES-EVs or CTRL-EVs for 7 days. (Figure 3A)

Results showed that human brain organoid area enlarges ~ 1.5 times following ES-EVs compared to CTRL-EVs treatment for one week. (Figure 3B) Data are analyzed from n=10 organoids per condition.

Moreover, the percentage of cells positive for the proliferation marker Ki76 and for PHH3, the mitosis marker, significantly increased in organoids treated with ES-EVs compared to those treated with CTRL-EVs. (Figure 3C and 3D) Data are analyzed from n=8-9 organoids per condition.

This result strongly indicates that the cargo of EVs has been modified with the electrical stimulation method of the present invention. ES-EVs effectively accelerates the growth of human brain organoids, showing that ES-EVs are beneficial for developing in vitro 3D models and for generating therapeutic EVs for the treatment of the central nervous system.

### Example 2. Comparative studies with electroactive cells

### Example 2. 1. Human neuroblastoma

Low voltage low frequency electrical stimulation of human neuroblastoma cells (SH-SY5Y) also amplifies the concentration of produced extracellular vesicles and modifies their cargo towards pro-regenerative content.

SH-SY5Y cells were cultured (30K cell/cm²) in electrical stimulation chamber and electrical stimulation protocols were applied in the conditions described in Table 2. All groups treated for 1 hour per day, for 3 days. The control group kept in culture for 3 days with no treatment. All experiments have been carried out at least in three biological replicates.

| **Table 2.** | | | | | | |
|---|---|---|---|---|---|---|
| **Regime** | **Graph** | **Frequency** | **Amplitude** | **Field** | **Offset** | **Pulse width** |
| ES1 | 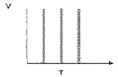 | 1 Hz | 800 mV | ~25 mV/mm | 0 | 500 µs |
| ES2 | 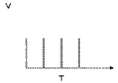 | 1 Hz | 400 mV | ~12.5 mV/mm | 0 | 500 µs |
| ES3 | 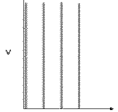 | 1 Hz | 1.6 V | ∼ 50 mV/mm | 0 | 500 µs |
| ES4 | 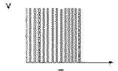 ' | 500 Hz | 800 mV | ~25 mV/mm | 0 | 500 µs |
| ES5 | 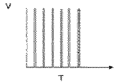 | 50 Hz | 800 mV | ~25 mV/mm | 0 | 500 µs |
| ES6 | 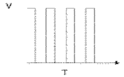 | 1 Hz | 800 mV | ~25 mV/mm | 0 | 250 ms |
| ES7 | 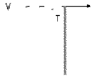 | 1 Hz | 800 mV | ~25 mV/mm | 0 | 500 µs |

Conditioned medium (secretome) were collected from each replicate and centrifuged with 300xg for 10 min to remove the dead cells debris. The supernatant centrifuged 2000xg for 20 min to remove apoptotic bodies. The purified secretome were subjected to nano particle tracking analysis (NTA). Three technical replicates have been used for nanoparticle tracking analysis of EVs. The particle size distribution and the number of particles in the collected EVs suspension were measured using NTA device (Malvern Panalytical NanoSight NS300 Instrument, UK).

Figure 4. Electrical stimulation amplifies EVs production in human Neuroblastoma. A) Effect of different electrical stimulation regimes (see table 2) on EVs concentration produced by SH-SY5Y neuroblastoma cells. B) Mean size of EVs secreted from electrically stimulated SH-SY5Y cells with different protocols (ES) and control (NO ES). C) Size distribution of EVs secreted from neuroblastoma cells treated with different electrical stimulation protocols and control. D) Transmission electron microscopy (TEM) images from EVs secreted from electrically stimulated SH-SY5Y cells (right) and control (left).

Particle concentration measurements show that electrical stimulation in general increases the amount of secreted EVs from the cells compared to control (No ES). The protocol in which cells were stimulated with 50 Hz pulsed signals, in the presence of 25 mV/mm electrical field, showed 3-to-5-fold increase in the number of particles per ml. Interestingly, it is demonstrated that parameters of electricals stimulation protocol, in particular frequency and pulse width, can tune the EVs secretion. (Figure 4A)

The size distribution and size determination results show that electrical stimulation does not change the size distribution of produced EVs and it is comparable with EVs naturally produced by these cells. (Figures 4A and 4 B)

TEM (JEM1010, Jeol) images confirmed that the morphology of the EVs has not been changed, meaning that the rise in the concentration is not due to a possible fragmentation of the EVs. (Figure 4D)

A discovery proteomics on the EVs collected from the electrically stimulated cells compared with EVs from cells treated with BDNF was also run. NanoLC Ultra system (Eksigent) directly coupled with an LTQ Orbitrap Velos instrument (Thermo Fisher Scientific) via nanoESI (ProxeonBiosystem) was used for this analysis.

Figure 5. Proteomics analysis of EVs obtained from electrical stimulated SH-SY5Y cells compared to EVs obtained from BDNF treated cells. Several groups of proteins in EVs obtained from electrical stimulated SH-SY5Y cells are upregulated compared to EVs from the cells treated by commercial BDNF. Checker pattern indicates group of protein that are involved in neurite growth, synaptic formation, memory, learning, and plasticity. White indicates protein involved in cancer suppression. Black indicates group of proteins that are anti-inflammatory and immunomodulators, promoting the survival after damage and oxidative stress. Gray indicates proteins with various functions. Overall, the results suggest that the content of EVs from electrically stimulated cells are pro-regenerative and pro-survival.

Results obtained from proteomics suggested that at least 7 proteins (indicated in checker pattern) with the main function of acceleration on neurite sprouting and maintenance and function of synapsis, which have roles in memory, learning and plasticity, have been significantly upregulated. Among those, Syntaxin-binding protein 3 (STXBP3) expressed extremely higher in ES-EVs. STXBP3 plays important role in the process of synaptic vesicle fusion and neurotransmitter release in the brain. It interacts with syntaxin proteins to regulate the docking and fusion of synaptic vesicles with the presynaptic membrane. STXBP3 is involved in the regulation of synaptic plasticity and the maintenance of proper neuronal function. (Figure 5)

Other group of proteins that expressed significantly higher in ES-EVs versus BDNF treated are immunomodulator proteins. These group of proteins, which are indicated in black, are involved in promoting survival of neurons after damage or oxidative stress, preventing excessive inflammation.

Overall, the results suggest that the content of EVs from electrically stimulated cells are pro-regenerative and pro-survival.

### Example 2. 2. Human Cardiac Organoids

It is also showed herein that low voltage electrical stimulation can be adapted to organoid culture to amplify the production of EVs.

750K primary human cardiac fibroblasts are mixed with bovine collagen to generate ring-shaped flexible cardiac organoids inside myriamed^{®} 48-well plates.

After one-week maturation, organoids were stimulated with 40 mV/mm, 500µs width pulses, with frequency of 1 Hz for 1 hour per day for 3 days. The secretome of three individual samples was collected and purified to remove dead cells and apoptotic bodies. Next, the supernatant was centrifuged for 2 hours with 100K xg. The pellet was collected (EVs) and resuspended in PBS and subjected to NTA to determine EVs concentration and size distribution.

Figure 6. Electrical stimulation amplifies EVs production in cardiac organoids. A) A single well of the 48-well myriamed^{®} plate equipped with mini silicon poles to culture the cardiac organoid (down). Human cardiac organoid developed from primary fibroblasts to study 3D cardiac fibrotic tissue in vitro (up). B) Average concentration of EVs secreted from the cardiac organoids with and without electrical stimulation. C) Size distribution of EVs secreted from cardiac organoids with and without electrical stimulation treatment. D) Mean size of EVs secreted from electrically stimulated and control (NO ES) organoids.

Results indicated that electrical stimulation increases the yield of EVs more than 2 folds in human cardiac organoids (Figure 6B), while the EVs size distribution and mean size remains similar to naturally produce EVS (Figures 6C and 6D).

## Claims

1. A method for the production of therapeutic extracellular vesicles from Mesenchymal Stem Cells comprising a step (a) of applying a direct pulsed electrical stimulation directly to the cells during a period of 30 minutes to 8 hours per 1 to 10 days with an electrical stimulation device comprising a plurality of metallic electrodes,
the Mesenchymal Stem Cells located in the cell culture wells of the electrical stimulation device, said cell culture wells comprising cell culture medium,
the plurality of metallic electrodes located inside the cell culture wells of the electrical stimulation device and in direct contact with the Mesenchymal Stem Cells and/or the cell culture medium,
the electrical stimulation device producing
• an electric field of between 10 mV/mm and 100 mV/mm
• a pulse of an amplitude range between 200 mV and 3 V,
• and a frequency ranging between 1 Hz and 1000 Hz,
wherein the pulse width is ranging between 1% and 50% of the duration of the pulse and wherein the duration of the pulse is the inverse of the frequency.

2. The method according to claim 1, wherein the metallic electrode is a platinum electrode.

3. The method according to any one of claims 1 or 2, wherein the direct pulsed electrical stimulation of step (a) is applied directly to the Mesenchymal Stem Cells during 30 minutes and 90 minutes per 1-10 days, preferably during 1 hour per day for 3 days.

4. The method according to any one of claims 1 to 3, wherein the electrical stimulation device produces a 50 Hz pulse width of at least 500us or a 1 Hz pulse width of at least 250 ms.

5. The method according to any one of claims 1 to 4, which further comprises a step (b), after step (a), of collecting and harvesting the Extracellular Vesicles produced in step (a), preferably by means of at least centrifugation, more preferably by means of centrifugation and filtration.

6. The method according to claim 5, wherein the collecting and harvesting the Extracellular Vesicles of step (b) includes ultra-centrifugation, ultrafiltration, density gradient ultrafiltration, hydrostatic dialysis, size-exclusion chromatography, and precipitation with polymer or protamine.

7. The method according to any one of claims 1 to 6, wherein the Mesenchymal Stem Cells are adipose MSCs, umbilical cord MSCs or bone marrow MSCs.

8. EVs obtained by the method according to any one of claims 1 to 7, or a composition comprising the EVs obtained by the method according to any one of claims 1 to 7, a kit comprising the EVs obtained by the method according to any one of claims 1 to 7, or a scaffold comprising the EVs obtained by the method according to any one of claims 1 to 7.

9. The EVs, the composition, or the kit according to claim 8, for use as a medicament, or for use in regenerative therapy or in the delivery of drugs.
